# EUROPÄISCHE PATENTANMELDUNG

(11) **EP 4 275 531 A1**
(43) Veröffentlichungstag der Anmeldung: **15.11.2023**
(21) Anmeldenummer: 23154247.3
(22) Anmeldetag: 31.01.2023
(51) Int. Cl.: A41C 1/10, A61F 5/02

(54) **SCHWANGERSCHAFTSMIEDER**

(30) Priorität: 10.05.2022 AT 1022022
(71) Anmelder: Volkert, Olaf, 4020 Linz (AT)
(72) Erfinder: Volkert, Olaf, 4020 Linz (AT)
(74) Vertreter: Hübscher & Partner Patentanwälte GmbH

(57) **Zusammenfassung**

Es wird ein Schwangerschaftsmieder beschrieben, das ein Bauchteil (7), sowie ein mit diesem verbindbares Rückenteil (1) umfasst, welches einen Wirbelsäulenabschnitt (2) aufweist, an dem beidseits je ein Lateralabschnitt (3) anschließt, der jeweils an seinem dem Wirbelsäulenabschnitt (2) gegenüberliegenden freien Ende über Befestigungsmittel (5, 6) lösbar mit dem Bauchteil (7) verbindbar ist. Erfindungsgemäß wird vorgeschlagen, dass in transversaler Richtung einerseits der Wirbelsäulenabschnitt (2) zugfest und andererseits die Lateralabschnitte (3) elastisch ausgebildet sind, und dass am Wirbelsäulenabschnitt (2) Gurtbänder (9, 10, 11, 12) angesetzt sind, die einander sowohl paarweise bezüglich des Wirbelsäulenabschnitts (2) gegenüberliegen als auch paarweise in longitudinaler Richtung benachbart sind, wobei die Gurtbänder (9, 10, 11, 12) unter Ausbildung eines Vorderbauchspanngurtes (13) sowie eines Unterbauchspanngurtes (14) mit einem auf den elastischen Bauchdeckenabschnitt (16) des Bauchteils (7) aufgebrachten, in transversaler Richtung zugfest ausgebildeten Stützabschnitt (15) verbindbar sind.

## Beschreibung

Die Erfindung bezieht sich auf ein Schwangerschaftsmieder, mit einem Bauchteil, das einen sowohl in longitudinaler als auch in transversaler Richtung elastischen Bauchdeckenabschnitt aufweist, und mit einem Rückenteil, das einen Wirbelsäulenabschnitt aufweist, an dem beidseits je ein Lateralabschnitt anschließt, der jeweils an seinem dem Wirbelsäulenabschnitt gegenüberliegenden freien Ende über Befestigungsmittel lösbar mit dem Bauchteil verbindbar ist.

Vorbekannte Schwangerschaftsmieder, d.h. Mieder für den Einsatz während der Schwangerschaft, weisen üblicherweise ein Rückenteil auf, welches den Rumpf der Schwangeren körperumfangsseitig ca. zu zwei Drittel umfasst und mit einem Bauchteil über entsprechende Verbindungsmittel lösbar verbunden werden kann. Derartige Schwangerschaftsmieder erfüllen durch die einstellbare, auf den Rumpf wirkende Grundkompression in erste Linie eine Stützfunktion, insbesondere zur Unterstützung der Wirbelsäule.

Darüber hinaus existieren für den Einsatz während der Schwangerschaft auch Beckenstütz- bzw. Symphysengurte (US 2009142988 A1), die im Wesentlichen einer schwangerschaftsbedingten Symphysenlockerung Abhilfe verschaffen.

Nachteilig an den vorbekannten Schwangerschaftsmiedern und Beckenstützgurten ist allerdings, dass diese nicht dazu geeignet sind, einer Rektusdiastase entgegenzuwirken, die bei den Betroffenen infolge der schwangerschaftsbedingten Bauchdeckenaufweitung regelmäßig auftritt. Ebenso wenig können derartige Schwangerschaftsmieder oder Beckenstützgurte eine das Hineingleiten in den Geburtskanal erleichternde Lagebeeinflussung des ungeborenen Kindes im Mutterleib bewirken, oder eine bereits vorhandene Kopflage des Kindes so stabilisieren, dass sich dieses in der Regel nicht mehr in eine für den Geburtsvorgang ungünstige Lage zurückdrehen kann.

Ferne sind auch Mieder zur postpartalen Anwendung bekannt (DE1852007U), die eine Rückbildung des Leibes unterstützen sollen. Um zu diesem Zweck einen besonders starken Zug bzw. eine starke Kompressionswirkung zu erreichen, weisen derartige postpartale Mieder sich über der Bauchdecke kreuzende Züge auf. Abgesehen davon, dass solche Mieder konstruktionsbedingt einer Symphysenlockerung nicht sinnvoll entgegenwirken können, sind jene Mieder grundsätzlich nicht für den Einsatz während der Schwangerschaft geeignet, zumal die starken Zug- und Kompressionskräfte auch eine Gefahr für das ungeborene Kind bergen würden.

Es besteht somit ein Bedarf an einem Schwangerschaftsmieder der eingangs geschilderten Art, mit welchem einerseits sowohl eine Unterstützung und Entlastung der Wirbelsäule und des Beckenrings der Schwangere erfolgt, als auch einer Rektusdiastase zuverlässig entgegengewirkt wird, und andererseits eine den Geburtsvorgang erleichternde Lagebeeinflussung bzw. Lagestabilisierung des ungeborenen Kindes ermöglicht wird, ohne die Gesundheit sowohl der Schwangeren als auch des Kindes zu gefährden.

Die Erfindung löst die gestellte Aufgabe dadurch, dass wenigstens in transversaler Richtung einerseits der Wirbelsäulenabschnitt zugfest und andererseits die Lateralabschnitte elastisch ausgebildet sind, und dass am Wirbelsäulenabschnitt Gurtbänder angesetzt sind, die einander sowohl paarweise bezüglich des Wirbelsäulenabschnitts gegenüberliegen als auch paarweise in longitudinaler Richtung benachbart sind, wobei die Gurtbänder unter Ausbildung eines Vorderbauchspanngurtes einerseits sowie eines Unterbauchspanngurtes andererseits mit einem auf den elastischen Bauchdeckenabschnitt des Bauchteils aufgebrachten, wenigstens in transversaler Richtung zugfest ausgebildeten Stützabschnitt verbindbar sind. Zufolge dieser Merkmale wird, zusätzlich zu der über das Bauch- und Rückenteil bewirkten Grundkompression des Schwangerenrumpfes sowie der mithilfe des Unterbauchspanngurtes bewirkten Unterstützung und Entlastung der Wirbelsäule, des Beckenrings, des Unterbauches und des Beckenbodens auch eine nachhaltige Entlastung der Bauchmuskulatur, insbesondere der geraden Bauchmuskeln (m. rectus abdominis), durch den Vorderbauchspanngurt ermöglicht. Erfindungsgemäß ergeben sich dabei sowohl günstige Kompressionsbedingungen als auch eine vorteilhafte Stützung der Wirbelsäule dadurch, dass wenigstens in transversaler Richtung einerseits der Wirbelsäulenabschnitt zugfest und andererseits die Lateralabschnitte elastisch ausgebildet sind. Im Sinne der Erfindung wird unter der transversalen Richtung jene Richtung verstanden, die im Wesentlichen in Umfangsrichtung des Mieders bzw. im getragenen Zustand in Körperumfangsrichtung der Schwangeren verläuft. Darüber hinaus bewirkt die Zugrichtung des Unterbauchspanngurtes vom Schambein/Unterbauch seitlich um das Darmbein herum in Richtung der Iliosakralgelenke eine Stabilisierung des Beckenrings, eine Stützung des Unterbauches sowie eine zusätzliche Entlastung des Beckenbodens. Anhand der erfindungsgemäßen Merkmale hat sich außerdem überraschenderweise gezeigt, dass die Kombination aus Vorder- und Unterbauchspanngurt nicht nur die Anhebung des Unterbauches sowie die Entlastung und Unterstützung des Beckens und der Wirbelsäule noch weiter verbessert, sondern dass darüber hinaus der Vorderbauchspanngurt auch einer Rektusdiastase entgegenwirkt. Dadurch, dass der Vorderbauchspanngurt einen rückwärtigen, also zur Körpermitte der Schwangeren hin gerichteten Zug auf die Vorausstülpung der Bauchmitte erzeugt, wird die Ausdehnung des Bauchanteiles nach ventral, also weiter nach außen, limitiert. Folglich kann so die übermäßige, typischerweise durch die schwangerschaftsbedingte Bauchdeckenaufweitung ausgelöste Zugbelastung auf die geraden Bauchmuskeln verhindert werden, die ungehindert ansonsten eine Rektusdiastase begünstigen bzw. zwangsläufig zu einer Rektusdiastase führen würde. Darüber hinaus hat sich gezeigt, dass durch das Vorsehen eines erfindungsgemäßen Vorderbauchspanngurtes und der damit verbundenen Begrenzung des Bauches bzw. dem damit verbundenen Rückhalten des Bauches eine Veränderung der Lage des ungeborenen Kindes erreicht werden kann, ohne dass es zu einer gesundheitlichen Gefährdung des Kindes kommt. Um diese erfindungsgemäße Wirkung des Vorderbauchspanngurtes weiter zu verbessern, werden vorzugsweise die den Vorderbauchspanngurt bildenden Gurtbänder sowie die mit diesen verbindbaren, am Bauchteil vorgesehenen Stützabschnitte so angeordnet, dass bei der Benutzung des erfindungsgemäßen Schwangerschaftsmieders der obere Rand des Vorderbauchspanngurtes in Bezug auf den Schwangerenbauch im Bereich des Nabels, insbesondere knapp oberhalb bzw. knapp unterhalb des Nabels, entlang verläuft. Insbesondere wird dadurch eine zu weite Frontallage verhindert und in weiterer Folge das Kind in einen günstigen Geburtswinkel geführt, sodass dieses in Kopflage problemlos in den Geburtskanal hineingeleiten kann. Ebenso kann nach bereits erfolgter Wendung des ungeborenen Kindes aus der Steißlage in die Kopflage der Bauchraum der Schwangeren zufolge der durch das erfindungsgemäße Schwangerschaftsmieder wirkenden Zug- und Druckkräfte derart in Form gehalten werden, dass das Kind bis zur Geburt in der Kopflage stabilisiert wird und sich nicht ohne weiteres wieder von selbst in die Steißlage zurückdrehen kann. Die Stabilisierung des ungeborenen Kindes im i Kopflage ist dabei wiederum entscheidend für eine weitere Entlastung der Bauchmuskulatur und des Beckenbodens der Schwangeren

Grundsätzlich kann die Anzahl der Gurtbänder so gewählt werden, sich damit Gurtbänderpaare bilden lassen, die jeweils dem Unterbauchspanngurt bzw. dem Vorderbauchspanngurt zuzuordnen sind. Beispielsweise kann der Unterbauchspanngurt zwei Gurtbänderpaare, also insgesamt vier Gurtbänder, umfassen, wohingegen der Vorderbauchspanngurt lediglich ein Gurtbänderpaar, d.h. insgesamt zwei Gurtbänder aufweist. Besonders günstige konstruktive Bedingungen ergeben sich allerdings, wenn genau vier Gurtbänder vorgesehen sind, wobei je zwei der Gurtbänder dem Vorderbauchspanngurt und die jeweils anderen zwei Gurtbänder dem Unterbauchspanngurt zugeordnet sind. Die Gurtbänder können für sich jeweils gesondert am Wirbelsäulenabschnitt angesetzt sein. Es ist aber ebenso möglich, dass ein Gurtband des Vorderbauchspanngurtes und ein Gurtband des Unterbauchspanngurtes, welche in Bezug auf den Wirbelsäulenabschnitt auf derselben Miederseite angeordnet sind, in eine gemeinsame Ansatzbasis münden bzw. zusammenlaufen, wobei dann diese gemeinsame Ansatzbasis am Wirbelsäulenabschnitt angesetzt ist.

Obwohl bereits eine Stützwirkung durch den erfindungsgemäßen Wirbelsäulenabschnitt erfolgt, empfiehlt es sich, dass zur Aufnahme der durch die Gurtbänder in den Wirbelsäulenabschnitt eingeleiteten Zugkräfte am Wirbelsäulenschnitt im jeweiligen Ansatzbereich der Gurtbänder je ein in longitudinaler Richtung verlaufendes Verstärkungselement, insbesondere ein Verstärkungsstab, eingearbeitet ist. Die longitudinale Richtung entspricht im Wesentlichen der quer zur Umfangsrichtung verlaufenden Längsrichtung des Schwangerschaftsmieders bzw. im getragenen Zustand der Körperlängsachsenrichtung der Schwangeren. Die Verstärkungselemente bilden folglich gemeinsam mit dem Wirbelsäulenabschnitt ein vorteilhaftes Widerlager für die an den Stützabschnitten des Bauchteils befestigbaren und dadurch spannbaren Gurtbänder, und ermöglichen somit gemeinsam mit dem Wirbelsäulenabschnitt eine Stützfunktion bzw. sind Gegenhalt für die durch die Gurte eingeleiteten Zugkräfte. Die Ausrichtung, Formgebung sowie Materialwahl der Verstärkungselemente ist dergestalt, dass einerseits die Verstärkungselemente samt Wirbelsäulenabschnitt eine Anlage im Gesäßbereich und im rückenseitigen Brustkorbbereich der Schwangeren bilden, und dass andererseits aufgrund der in die Verstärkungselemente eingeleiteten Zugkräfte eine Flexionswirkung in der Lendenwirbelsäule erzielt und folglich eine Entlordosierung derselben begünstigt wird.

Vorteilhafte Spannbedingungen in Bezug auf den Unter- und Vorderbauchspanngurt ergeben sich, wenn an den jeweils freien Enden der Gurtbänder je eine Klettverschlussschlaufe angebracht ist, der je ein am Stützabschnitt des Bauchteils angebrachter Spannbügel zugeordnet ist. Der Spannbügel kann insbesondere eine Spannöse bilden, die beispielsweise über eine entsprechende Schlaufe am Stützabschnitt befestigt ist.

Um sowohl eine stabile Formgebung zu ermöglichen, als auch eine vorteilhafte Grundkompression auf den Rumpf der Schwangeren aufzubringen, sodass eine Überdehnung der Haut und des Bindegewebes vermindert werden kann, wird vorgeschlagen, dass der Lateralabschnitt aus einem Textil gefertigt ist, welches eine Zusammensetzung aus 50 - 80 %, bevorzugt 60 - 70 %, insbesondere 65 % Polyamidfasern, sowie aus 20 - 50 %, bevorzugt, 30 - 40 %, insbesondere 35 % Elasthanfasern aufweist. Der Bauchteil kann insbesondere an seinen in transversaler Richtung beidseits an den Bauchdeckenabschnitt anschließenden Endabschnitten ebenfalls aus demselben Textil wie die Lateralabschnitte des Rückenteils gefertigt sein. Vorzugsweise ist das Textil ein Lycra-Gewebe.

Um eine ausreichende, dabei aber möglichst hautschonende Grundkompression im Bereich der Bauchdecke der Schwangeren zu erreichen, empfiehlt es sich, dass der Bauchdeckenabschnitt des Bauchteils aus einem Textil gefertigt ist, welches eine Zusammensetzung aus 60 % Viskosefasern, 35 % Polyamidfasern, sowie 5 %, Elasthanfasern aufweist. Vorzugsweise ist das Bauchteil aus handelsüblich bekanntem Jersey gefertigt.

Günstige Kompressions- und Formgebungsbedingungen bei einem für die Schwangere angenehmen Tragegefühl können dadurch erzielt werden, dass der Bauchdeckenabschnitt in transversaler Richtung eine im Wesentlichen konvexe Außenkontur ausbildet. Dies bedeutet, dass in Bezug auf die Bauchdeckenmitte die Außenkontur des Bauchdeckenabschnittes in Körperumfangsrichtung radial nach außen hin ausgebogen ist.

Um die Stützwirkung des Rückenteils weiter zu verbessern, kann der jeweilige Lateralabschnitt in transversaler Richtung durch je wenigstens einen Verstärkungsabschnitt unterbrochen sein, der wenigstens in transversaler Richtung zugfest ausgebildet ist. Vorzugsweise ist der wenigstens eine Verstärkungsabschnitt aus einem Baumwolltextil, insbesondere einem Drellstoff aus Baumwolle, gefertigt.

Es versteht sich von selbst, dass je nach Anforderung der Wirbelsäulenabschnitt, die Lateralabschnitte, die Verstärkungsabschnitte und/oder die diversen Abschnitte des Bauchteils jeweils auch aus mehreren Textillagen aufgebaut sein kann. Die einzelnen Textillagen eines jeweiligen Abschnitts können insbesondere miteinander vernäht sein.

Um den Tragekomfort zu erhöhen und insbesondere ein problemloses Sitzen zu ermöglichen, wird vorgeschlagen, dass das Rückenteil so zugeschnitten ist, dass dieses im getragenen Zustand des Schwangerschaftsmieders im longitudinal oberen Bereich unterhalb der Schulterblätter, sowie im longitudinal unteren Bereich oberhalb des Steißbeinansatzes abschließt.

In der Zeichnung ist der Erfindungsgegenstand beispielsweise dargestellt. Es zeigen
- Fig. 1: eine schematische Rückansicht eines erfindungsgemäßen Schwangerschaftsmieders mit offenen Gurtbändern,
- Fig. 2: eine der Fig. 1 entsprechende Vorderansicht und
- Fig. 3: eine der Fig. 1 und 2 entsprechende Seitenansicht bei geschlossenen Gurtbändern.

Ein erfindungsgemäßes Schwangerschaftsmieder umfasst ein Rückenteil 1, das einen Wirbelsäulenabschnitt 2 aufweist. Am Wirbelsäulenabschnitt 2 schließt beidseits je ein Lateralabschnitt 3 an. Darüber hinaus ist der jeweilige Lateralabschnitt 3 in transversaler Richtung durch je einen Verstärkungsabschnitt 4 unterbrochen. Dadurch wird der jeweilige Lateralabschnitt 3 in zwei Unterabschnitte geteilt, die sich somit in transversaler Richtung beidseits des mit diesen Unterabschnitten vernähten Verstärkungsabschnitts 4 befinden.

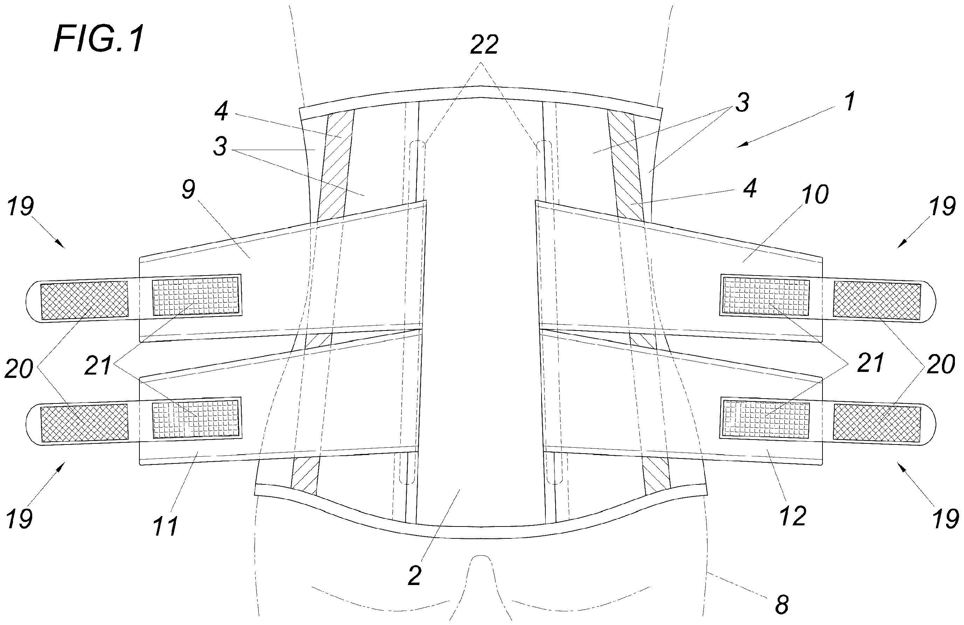

Wie dies insbesondere aus Fig. 1 hervorgeht, ist das Rückenteil 1 so zugeschnitten, dass dieses im getragenen Zustand des Schwangerschaftsmieders im longitudinal oberen Bereich unterhalb der Schulterblätter, sowie im longitudinal unteren Bereich oberhalb des Steißbeinansatzes abschließt.

Der Wirbelsäulenabschnitt 2 sowie die Verstärkungsabschnitte 4 sind jeweils sowohl in longitudinaler als auch in transversaler Richtung zugfest, wohingegen die Lateralabschnitte 3 sowohl in longitudinaler als auch in transversaler Richtung elastisch ausgebildet sind. Vorzugsweise ist der Wirbelsäulenabschnitt 2 aus einem doppelt gelegten Baumwollstoff gefertigt. Die Lateralabschnitte 3 sind hingegen bevorzugt aus Lycra-Gewebe gebildet. Die Verstärkungsabschnitte 4 sind wiederum insbesondere aus einem baumwollbasierten Drellstoff gefertigt.

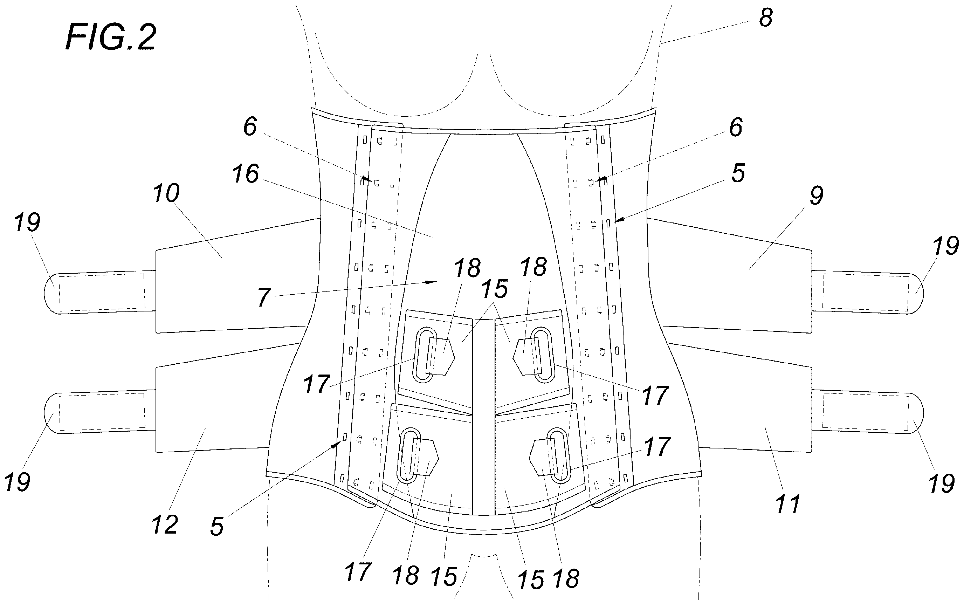

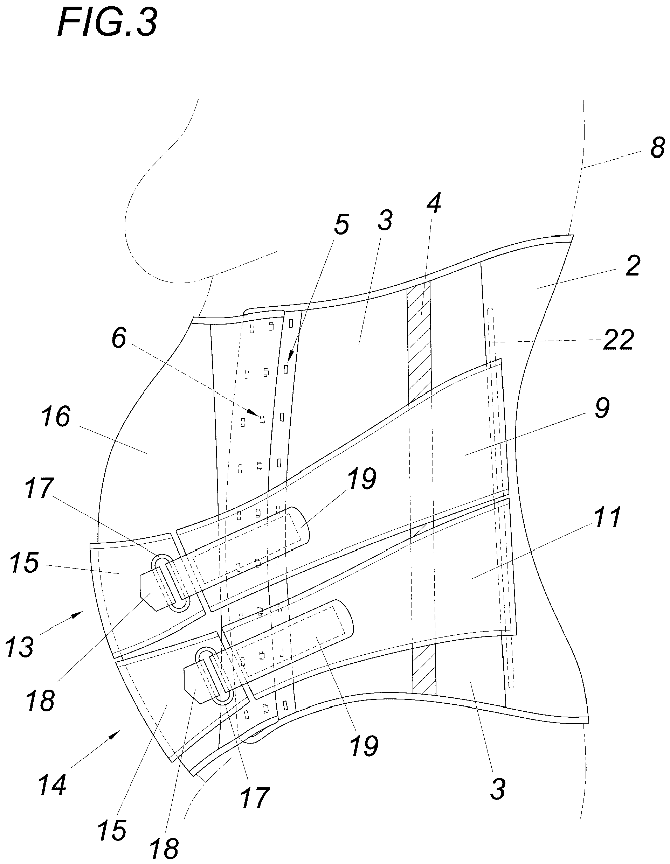

Das Rückenteil 1 ist an seinem dem Wirbelsäulenabschnitt 2 gegenüberliegenden freien Ende über Befestigungsmittel 5, 6 lösbar mit einem Bauchteil 7 verbindbar, wie dies aus den Fig. 2 und 3 hervorgeht. Als Befestigungsmittel 5 dienen im vorliegenden Ausführungsbeispiel an den freien Enden des Rückenteiles außenliegend aufgenähte, vorzugsweise mehrreihige Augenbänder. Diese korrespondieren mit den Befestigungsmitteln 6, die als an den freien Enden des Bauchteils 7 innenliegend aufgenähte, vorzugsweise einreihige Hakenbänder ausgebildet sind. Dadurch, dass einerseits die Augenbänder mehrreihig und andererseits die dazu korrespondierenden Hakenbänder einreihig ausgebildet sind, ist eine der Bauchform und -größe Rechnung tragende Einstellung der Befestigungsmittel 5, 6 und damit eine gezielte Einstellung der gewünschten Grundkompression auf den vom Schwangerschaftsmieder umfassten Rumpfbereich der Schwangeren 8 problemlos möglich. Es versteht sich von selbst, dass grundsätzlich auch die Hakenbänder mehrreihig und stattdessen die Augenbänder einreihig ausgebildet sein können. Ebenso ist es grundsätzlich möglich, dass die Augenbänder dem Bauchteil 7 und die Hakenbänder dem Rückenteil 1 zugeordnet sind. Grundsätzlich sind sämtliche Befestigungsmittel 5, 6 denkbar, die im Wesentlichen eine individuelle Anpassung der Grundkompression ermöglichen.

Am Wirbelsäulenabschnitt 2 sind vier Gurtbänder 9, 10, 11, 12 angesetzt, die einander sowohl paarweise bezüglich des Wirbelsäulenabschnitts 2 gegenüberliegen als auch paarweise in longitudinaler Richtung benachbart sind, wie dies aus Fig. 1 ersichtlich ist. Die Gurtbänder 9, 10 sind unter Ausbildung eines Vorderbauchspanngurtes 13 und die Gurtbänder 11, 12 unter Ausbildung eines Unterbauchspanngurtes 14 mit entsprechenden, wenigstens in transversaler Richtung zugfest ausgebildeten Stützabschnitten 15 verbindbar. Die Stützabschnitte 15 sind auf einem elastischen, insbesondere aus Jersey gefertigten Bauchdeckenabschnitt 16 des Bauchteils 7 aufgenäht. Auf den Stützabschnitten 15 sind außerdem Spannbügel 17 aufgebracht. Hierzu sind die jeweiligen Spannbügel 17 über jeweils entsprechende Schlaufen 18 am jeweiligen Stützabschnitt 15 befestigt. Um demnach die Gurtbänder 9, 10, 11, 12 unter Ausbildung des Vorderbauchspanngurtes 13 bzw. des Unterbauchspanngurtes 14 über die Spannbügel 17 mit den Stützabschnitten 15 zu verbinden, ist an den jeweils freien Enden der Gurtbänder 9, 10, 11, 12 je eine Klettverschlussschlaufe 19 angebracht. Wie dies insbesondere in Fig. 1 schematisch angedeutet wird, umfassen dabei die Klettverschlussschlaufen 19 jeweils einen Kletthakenabschnitt 20 sowie einen Flauschabschnitt 21.

Aus Verstärkungsgründen sind die Gurtbänder 9, 10, 11, 12, die jeweiligen Stützabschnitte 15, sowie auch das Schwangerschaftsmieder selbst im jeweiligen Randbereich zusätzlich mit einem Einfassband aus Drellstoff versehen, wie dies in den Zeichnungen schematisch angedeutet wird.

Zur verbesserten Aufnahme der durch die Gurtbänder 9, 10, 11, 12 in den Wirbelsäulenabschnitt 2 eingeleiteten Zugkräfte ist am Wirbelsäulenschnitt 2 im jeweiligen Ansatzbereich der Gurtbänder 9, 10, 11, 12 je ein in longitudinaler Richtung verlaufendes Verstärkungselement 22, im vorliegende Fall ein Verstärkungsstab, eingearbeitet. Um bei ausreichender Stabilität auch einen entsprechenden Tragekomfort zu bieten sowie insbesondere ein Sitzen zu erleichtern, reichen die Verstärkungselemente 22 in longitudinaler Richtung jeweils bis ca. 2cm unter den oberen bzw. unteren Miederrand.

Für günstige Kompressions- und Formgebungsbedingungen bei einem für die Schwangere 8 angenehmen Tragegefühl bildet der Bauchdeckenabschnitt 16 in transversaler Richtung eine im Wesentlichen konvexe Außenkontur aus, wie dies insbesondere aus Fig. 2 hervorgeht. Dies bedeutet, dass in Bezug auf die Bauchdeckenmitte die Außenkontur des Bauchdeckenabschnittes 17 in transversaler Richtung, d.h. in Körperumfangsrichtung, radial nach außen hin ausgebogen ist. Die insbesondere in transversaler Richtung beidseits an den Bauchdeckenabschnitt 16 anschließenden Endabschnitte des Bauchteils 7, auf welchen auch die Befestigungsmittel 6 aufgebracht sind, können wie die Lateralabschnitte 3 ebenfalls aus Lycra-Gewebe gefertigt sein.

Aufgrund der erfindungsgemäßen Kombinationswirkung aus Vorderbauchspanngurt 13, Unterbauchspanngurt 14 sowie des über Befestigungsmittel 5, 6 miteinander verbinden Rückenteiles 1 und Bauchteiles 7 wird in Summe nicht nur eine Unterstützung und Entlastung der Wirbelsäule und des Beckenrings erreicht, sondern darüber hinaus sowohl einer Rektusdiastase zuverlässig entgegengewirkt, als auch eine den Geburtsvorgang erleichternde Lagebeeinflussung bzw. Lagestabilisierung des ungeborenen Kindes ermöglicht.

## Patentansprüche

1. Schwangerschaftsmieder, mit einem Bauchteil (7), das einen sowohl in longitudinaler als auch in transversaler Richtung elastischen Bauchdeckenabschnitt (16) aufweist, und mit einem Rückenteil (1), das einen Wirbelsäulenabschnitt (2) aufweist, an dem beidseits je ein Lateralabschnitt (3) anschließt, der jeweils an seinem dem Wirbelsäulenabschnitt (2) gegenüberliegenden freien Ende über Befestigungsmittel (5, 6) lösbar mit dem Bauchteil (7) verbindbar ist, **dadurch gekennzeichnet, dass** wenigstens in transversaler Richtung einerseits der Wirbelsäulenabschnitt (2) zugfest und andererseits die Lateralabschnitte (3) elastisch ausgebildet sind, und dass am Wirbelsäulenabschnitt (2) Gurtbänder (9, 10, 11, 12) angesetzt sind, die einander sowohl paarweise bezüglich des Wirbelsäulenabschnitts (2) gegenüberliegen als auch paarweise in longitudinaler Richtung benachbart sind, wobei die Gurtbänder (9, 10, 11, 12) unter Ausbildung eines Vorderbauchspanngurtes (13) einerseits sowie eines Unterbauchspanngurtes (14) andererseits mit einem auf den elastischen Bauchdeckenabschnitt (16) des Bauchteils (7) aufgebrachten, wenigstens in transversaler Richtung zugfest ausgebildeten Stützabschnitt (15) verbindbar sind.

2. Schwangerschaftsmieder nach Anspruch 1, **dadurch gekennzeichnet, dass** genau vier Gurtbänder (9, 10, 11, 12) vorgesehen sind, wobei je zwei Gurtbänder (9, 10) dem Vorderbauchspanngurt (13) und die jeweils anderen zwei Gurtbänder (11, 12) dem Unterbauchspanngurt (14) zugeordnet sind.

3. Schwangerschaftsmieder nach Anspruch 1 oder 2, **dadurch gekennzeichnet, dass** zur Aufnahme der durch die Gurtbänder (9, 10, 11, 12) in den Wirbelsäulenabschnitt (2) eingeleiteten Zugkräfte am Wirbelsäulenschnitt (2) im Ansatzbereich der Gurtbänder (9, 10, 11, 12) je ein in longitudinaler Richtung verlaufendes Verstärkungselement (22), insbesondere ein Verstärkungsstab, eingearbeitet ist.

4. Schwangerschaftsmieder nach einem der Ansprüche 1 bis 3, **dadurch gekennzeichnet, dass** an den jeweils freien Enden der Gurtbänder (9, 10, 11, 12) je eine Klettverschlussschlaufe (19) angebracht ist, der je ein am Stützabschnitt (15) des Bauchteils (7) angebrachter Spannbügel (17) zugeordnet ist.

5. Schwangerschaftsmieder nach einem der Ansprüche 1 bis 4, **dadurch gekennzeichnet, dass** der Lateralabschnitt (3) aus einem Textil gefertigt ist, welches eine Zusammensetzung aus 50 - 80 %, bevorzugt 60 - 70 %, insbesondere 65 % Polyamidfasern, sowie aus 20 - 50 %, bevorzugt, 30 - 40 %, insbesondere 35 % Elasthanfasern aufweist.

6. Schwangerschaftsmieder nach einem der Ansprüche 1 bis 5, **dadurch gekennzeichnet, dass** der Bauchdeckenabschnitt (16) des Bauchteils (7) aus einem Textil gefertigt ist, welches eine Zusammensetzung aus 60 % Viskosefasern, 35 % Polyamidfasern, sowie 5 %, Elasthanfasern aufweist.

7. Schwangerschaftsmieder nach einem der Ansprüche 1 bis 6, **dadurch gekennzeichnet, dass** der Bauchdeckenabschnitt (16) in transversaler Richtung eine im Wesentlichen konvexe Außenkontur ausbildet.

8. Schwangerschaftsmieder einem der Ansprüche 1 bis 7, **dadurch gekennzeichnet, dass** der jeweilige Lateralabschnitt (3) in transversaler Richtung durch je wenigstens einen Verstärkungsabschnitt (4) unterbrochen ist, der wenigstens in transversaler Richtung zugfest ausgebildet ist.

9. Schwangerschaftsmieder nach einem der Ansprüche 1 bis 8, **dadurch gekennzeichnet, dass** das Rückenteil (1) so zugeschnitten ist, dass dieses im getragenen Zustand des Schwangerschaftsmieders im longitudinal oberen Bereich unterhalb der Schulterblätter, sowie im longitudinal unteren Bereich oberhalb des Steißbeinansatzes abschließt.
